# EUROPEAN PATENT APPLICATION

(11) **EP 1 616 570 A1**
(43) Date of publication of application: **18.01.2006**
(21) Application number: 04723795.3
(22) Date of filing: 26.03.2004
(51) Int. Cl.: A61K 31/715, A61P 13/12, A61P 1/16, A23L 1/308

(54) **FOOD FOR IMPROVING CLINICAL CONDITIONS CAPABLE OF LOWERING THE CONCENTRATION OF LOW-MOLECULAR WEIGHT NITROGEN-CONTAINING COMPOUNDS IN BLOOD**

(30) Priority: 26.03.2003 JP 2003086141
(71) Applicant: Cheiron Japan Co., Tokyo 156-0055 (JP)
(72) Inventor: YUGARI, Yasumi, 247 - 0053 (JP); KOBAYASHI, Takaaki, Kanagawa 2440803 (JP); FURUYA, Shu, Fukushima 9610835 (JP)
(74) Representative: Murphy, Colm Damien
(86) International application number: PCT/JP2004/004319
(87) International publication number: WO 2004/084919

(57) **Abstract**

The present invention aims at preventing or retarding a transition to hemodialysis treatment in kidney failure or decreasing the number of implementation of the hemodialysis therapy and preventing or lessening occurrence of accompanying hyperammonemia in hepatic insufficiency.

To accomplish the purposes described above, the pathological improvement food of the invention is featured by being composed chiefly of indigestible polysaccharides and having restricted addition of protein components.

Energy produced by exploiting the indigestible polysaccharides contributes to fungal protein synthesis using, as an N-source, urea or ammonia secretion into the intestine and excretion of proliferated bacteria with feces, so that nitrogen-containing low-molecular substances in the blood can be decreased, thereby to improve disorder of the kidney and liver.

## Description

### TECHNICAL FIELD

This invention relates to food suitable for improving clinical conditions of patients particularly with kidney failure or hepatic failure, and more particularly to pathological improvement food capable of being used for preventing or retarding a transition to hemodialysis treatment in kidney failure or decreasing the number of implementation of the hemodialysis therapy and preventing or lessening occurrence of accompanying hyperammonemia in hepatic insufficiency.

### BACKGROUND ART

There has been so far known that dietary fiber or nonfibrillary hydroscopic carbon has a function of increasing the excretion amount of nitrogen into feces without digesting dietary fiber or non-filxillary hydraulic carbon in the small intestine of the human or animal. This is disclosed in, for example, Sealock R. R., Basinski, D. H. & Murlin, J. R.: J. Nutrition, 22, 589-596 (1941); Mason, V C. & Palmer, R. M.: Proc. Nutr. Soc., 32, 82A, 83A(1973); Weber, F. L. Gastroenterology: 77, 518-523(1979); and Stephen, A., M. & Cummings, J.H.: Proc, Nutr. Soc., 38, 141A(1979).

An application example for applying the dietary fiber to renal disease is disclosed in Japanese Patent Application Publication HEI 02-101016A. This publication discloses preparation for improving renal disease, which contains the dietary fiber as an active ingredient made from the hull of edible seed. The publication suggests that the dietary fiber made from the hull is effective at prolonging the life of a rat with acute kidney failure resulting from renal ligation. However, since the case of acute renal disorder of renal ligation has no application to a chronic disorder case of a human and it is uncertain whether or not the dietary fiber is effective in the treatment of renal disease, various kinds of dietary fibers are dealt with inclusively in the publication. Thus, there is an undesirable possibility of side effects such as diarrhea due to dietary fiber intake increased simply.

For the meanwhile, it has known that 40% to 70% of nitrogen in the feces are derived from fungal protein within intestinal bacteria, which makes use of ammonia produced by decomposition of urea secreted into the intestine to convert the fungal protein. (See Wrong, O. M. & Vince, A.: Proc. Nutr. Soc., 43, pp.77-86(1984))

However, there has been no attempt to lowering concentration of urea in the blood in the presence of kidney failure by positively using the urea secreted into the intestine for of fungal protein synthesis of the intestinal bacteria in the intestine.

The medical guideline recommended by Japanese Society of Nephrology provides restriction of protein intake (0.5 to 0.6 g/kg body weight) for preventing increase of urea nitrogen in the blood (Blood Urea Nitrogen; BUN) of a renal failure patient ("Lifestyle guidance and dietary advice for patients with renal disease" compiled by Japanese Society of Nephrology). In addition to the restriction of protein intake, ingestion of quality protein having high biological value (such as animal protein, fishery product, whole egg etc.) is recommended.

To accomplish this purpose, low-gluten wheat is recommended as kidney failure diet, and therefore, there have been developed a good deal of low-protein food ofhigh-biological value. Further, various dietary supplements and elemental diets containing essential amino acid in a balanced manner have been devised and used

Although high-protein food of high-biological value is recommended for patients with hepatic failure, high-calorie elemental diets containing branched-chain amino acids (valine, leucine and isoleucine) have been devised and used for hepatic disease, because such amino acids provide promotion of insulin secretion and suppression of formation of tryptophan-derived serotonin or other brain amines by being antagonistic to tryptophan with respect to blood-brain barrier to prevent hepatic coma and cause awakening, and arginine contained in the amino acids activates urea cycle.

However, the aforesaid diets are mere food or enteral nutrient having a function of decreasing the supply of low-molecular-weight nitrogen-containing compounds such as urea and ammonia or raise hopes for producing symptomatic improvement through the provision of high-biological value protein or food composed of amino acid at an appropriate ratio of concentration designed in mixture. However, these diets do not fulfill the purpose and function of decreasing BUN since excretion of ingested nitrogen-containing compounds to the outside of the body through the route other than the principal metabolic excretion system or peripheral route.

As a result, when the low-molecular-weight nitrogen compounds such as urea derived from protein and amino acid in the diet are accumulated in the blood beyond the filtering ability of the kidney of a renal failure patient, the nitrogen compounds will be discharged outside ofthe body by hemodiafiltration.

Besides the dietetic treatment as noted above, there have been often used medicinal agents such as activated charcoal and cation-exchange resin for absorbing or inactivating ammonia drawn into the intestine to discharge or remove the ammonia as feces so as to decrease the blood ammonia level in the patient with hepatic failure.

There have been the following guidelines of alimentary therapy for accomplishing the purposes described above.
(1) Quantitative restriction and qualitative enrichment of protein:
   It is recommendable to quantitatively restrict the intake of protein and qualitatively enrich the quality of the protein in order for reducing the burden of nitric metabolite and preventing decrease in kidney function. In general, low-protein diets (0.6 g/kg body weight for kidney failure, and 12 to 1.5 g/kg body weight for hepatic failure) bring about an effect of slowing the deterioration in kidney failure or hepatic failure. Since the dietary treatment should extend over a long period of time, it is necessary to select the diets of high protein value (protein score) so as to regulate the balance of essential amino acids ("Lifestyle guidance and dietary advice for patients with renal disease" compiled by Japanese Society of Nephrology, Tokyoigakusha (1998), and "Nutritional diagnostics of liver diseases / Liver disease and nutritional therapy", Akiharu Watanabe and Misako Okita, Dai-ichi Shuppan Publishing Co., Ltd., pp.53-68 (1992)).
(2) Sufficient supply of dietary energy
   It is thought that 35 kcal/kg of daily energy is appropriate and daily energy intakes of 1800 to 2000 kcal are recommended. ("Lifestyle guidance and dietary advice for patients with renal disease" compiled by Japanese Society of Nephrology, Tokyoigakusha (1998), and "Nutritional diagnostics of liver diseases /Liver disease and nutritional therapy", Akiharu Watanabe and Misako Okita, Dai-ichi Shuppan Publishing Co., Ltd, pp.53-68 (1992))
(3) Sodium chloride (NaCl) should be restricted in a complication of anasarca and high-blood pressure.
(4) Potassium should be restricted in causing hyperkalemia
(5) Water intake should be restricted with salt when developing signs of anasarca, whereas dehydration requires attention in general.

It is generally important to satisfy the quantity and balance of required essential amino acids by low protein diets.

Under the conventional technology, it is necessary to give foods of high-biological value and having low total content of nitrogen to a patient with renal failure so as to reduce protein intake, which foods are prepared in such a manner that the proportion of better protein in the foods is increased for improving the quality of protein intake and reducing the total content of nitrogen or by using amino acids, especially, essential amino acid compounds having desirable relative proportions of the amino acids. However, when the concentration of low-molecular-weight nitrogen-containing compounds in the blood surpasses the renal filtration capability, the patient must undergo a hemodialysis treatment.

The hemodialysis treatment requires the patient to attend a hospital three or four times a week and have therapy three to five hours on attending oocasions. Large temporal, financial and psychological burdens will be exerted on the patient undergoing the hemodialysis treatment, resulting in a major detriment at the individual and social levels.

The medicine using the hemodialysis treatment for discharging the low-molecular-weight nitrogen-containing compounds from the blood will impose not only tremendous temporal, financial, physical and psychological burdens upon the patient, but also create more problems such as a large social burden of medical costs covered by a medical expense insurance. Such dialysis medical expenses will tend to be increasing from now on.

Hepatic coma in hepatic failure patients is caused by decrease in activity of urea cycle ofthe liver. Also, ammonia escalated in the blood due to the hepatic failure has a bearing on the circulation cycle of the intestine and blood. As a medical formula for discharging the ammonia to the outside of the body, there has been recognized a dose of chemical absorbing agent such as activated charcoal and cation-exchange resin is effective as a medical supply. However, heavy dosage of the absorbing agent having a possible side effect places an enormous burden to the patient. Under the circumstances, a need has been felt for an effective solution capable of reducing the burdens involved in treating.

Thus, the present invention seeks to provide a superior solution of the aforementioned problems involved in treating a patient with kidney failure or hepatic failure.

### DISCLOSURE OF THE INVENTION

The inventors of this invention has found the solution for attaining the objects described above, by administering indigestible polysaccharides as food having energy required for protein synthesis by bacteria, which is difficult to digest according to digestive enzyme and may be ingested as food according to intestinal bacteria, for the purpose of making use of low-molecular-weight nitrogen-containing compounds that are drained into the intestine and circulated in the reingested state as ingredients for fungal protein synthesis of intestinal bacteria to reduce the financial and temporal burdens involved in undergoing the hemodialysis treatment without taking any drug medicines having potential adverse effects. This invention could be achieved on the strength of this biochemical knowledge.

That is, the present invention provides pathological improvement food composed chiefly of indigestible polysaccharides and having restricted addition of protein components, much contains trace metal, vitamin and/or fat as required.

### BEST MODE FOR CARRYING OUT THE INVENTION

The invention will be described hereinafter in detail.

First, as to indigestible polysaccharides, starch and glycogen falling into polysaccharides are digested into glucose in the small intestine by digestive enzyme such as amylase in saliva or pancreatic fluid so as to be ingested into the body. Meanwhile, the polysaccharides typified by cellulose that is resistant to hydrolysis by the digestive enzyme have been known as dietary fiber (Dietary Fiber, Mipsley 1953). However, lactic acid bacilli and bifidobacteria that belong to intestinal normal inhabitants in the large intestine can exploit certain dietary fiber, and specifically, these enterobacteria can easily take in water-soluble dietary fiber (pectine, gum, oligosaccharide, alginic acid, chitosan, etc.) The dietary fiber exploited by the intestinal normal inhabitants is termed as indigestible polysaccharide. Polydextrose used in this invention is relative of oligosaccharide and falls into the indigestible polysaccharides belonging to the water-soluble dietary fiber.

Concretely speaking, the aforementioned indigestible polysaccharides include pectine, that is in an apple, strawberry and citrus, seaweed-derived alginic acid, fucoidan, crustacean chitin, chitosan, gums such as acacia gum, arum root-derived mannan, agar, polydextrose that is glucose polymer, testa-derived water-soluble dietary fiber (hemicellulose) such as corn, soybeans and psyllium, and sugar alcohol and polymers of sugar alcohol.

Of these polysaccharides, a compound made up of polydextrose and pectine has been used suitably. The proportion of the compound is preferably 0.05 to 100 parts by weight of pectine to 100 parts of polydextrose. The inventors have knowledge that such a compound brings about the most prominent effect of lowering BUN without causing diarrhea in ingestion thereof For unknown reasons, it is conceivable that the polydextrose and pectine are different in use-efliciency owing to intestinal bacteria such as lactic acid bacilli, consequently to further facilitate the activity of the lactic acid bacilli because of difference between them in metabolic rate.

Of the aforesaid substances, the pectine can easily be made from an apple or citrus fruit or obtained as a co-product in the making of citrus drinks.

There can be produced alginic acid and fucoidan from seaweeds, and the chitin and chitosan from crustacean shells with simple operation. The polydextrose can be made by polymerizing a natural product such as glucose as a basic ingredient in the presence of citric acid A variety of testa-derived water-soluble dietary fibers are commercially available.

The pathological improvement food according to the invention contains more than 5% by weight, preferably, 40% to 70% by weight of indigestible polysaccharide as a chief ingredient relative to the solid content of the food in term of dried foodstuff. When exceeding 70% by weight, the food is likely to be viscous, consequently to make food processing difficult.

The restriction of protein component in the food has a meaning of addition thereof within the range which does not raise blood urea nitrogen when ingesting with the indigestible polysaccharides and the protein content is 50% or less relative to the food of a kind. To be more specific, the proportion of the protein component (i.e. protein and its partially hydrolyzed protein (peptide)) to the solid content is made to 8% or less by weight, preferably about 2% by weight inteams of dried foodstuff.

The indigestible polysaccharides according to the invention contains, as basic ingredients, singly the aforementioned indigestible polysaccharides or compounds combined with several kinds of indigestible polysaccharides controlled in sweetness and acerbity with sweetener such as sucrose, fructose, glucose and sugar alcohol or natural sweetener such as luohan fruit essence and citric acid. To strengthen the effect of a dietary supplement, the indigestible polysaccharides of the invention may additionally contain sodium salt, potassium salt, magnesium salt and calcium salt, and further the needed volume of trace metals such as Zn, Fe, Cu, Co, Mn, Cr and Se in the form of the approved food additives. To further strengthen the effect of the dietary supplement, the indigestible polysaccharides of the invention may additionally contain, as a primary body, substances fortified with a suitable quantity of vitamins such as vitamin A, vitamin B family (e.g. B 1, B2, B6, B12 and nicotine acid), vitamin C, VitaminE, and folic acid, essential fatty acid or omega-3 fat. The indigestible polysaccharides of the invention may be produced in the form of a liquid (drink), jelly or cream or in a solid or semisolid form like a biscuit, cookie, bread, noodles, cake, ice cream, and sherbet and can be given singly or in combination to apadient with kidney failure or hepatic failure.

It may be preferable to make a variety of the indigestible polysaccharides according to the invention with variations so as to keep the patient from being bored by being flavored with fruit juice such as of an apple, lemon, strawberry, bluebeny, mandarin orange, orange, pomegranate, litchee, pineapple, papaya and banana, or flavoring agents.

Through experiments using pigs, it has been actually demonstrated the fact that the indigestible polysaccharides of the invention have a significant effect of reducing urinary excretion of nitrogen-containing low-molecular substances and excreting almost all the ingested nitrogen into faces (Lecture Abstract of 76th Meeting of The Japanese Society of Swine Science; Lecturers: Yamamoto et al., The Japanese Journal of Swine Science, Vol.38(4), p.227 (2001)). Yamamoto et al. announced in the meeting that urinary urea excretion was decreased to one-tenth when raise healthy pigs for seven days with a feed of low-protein feeding stuff (nitrogen intake 35.5 grams per day on an average) mixed with 30% by weight of apple juice lees (dried foodstuff). The nitrogen equivalent to the substance taken up by feces could be retrieved

The experimental results using the pigs support the possibility that the diet containing indigestible polysaccharides brings about significant effects of lowering the concentration of nitrogen-containing low-molecular substances in the blood of a hepatic failure patient so as to hold over treatments with hemodialysis or reduce the number of treatments with hemodialysis, or lowering the concentration of blood ammonia level of a kidney failure patient to prevent hepatic coma. However, the experimental results using the pigs do not always indicate the effectiveness of the pathological improvement food of the invention in treating the patients with kidney failure and hepatic failure from the viewpoint of the dose of the indigestible polysaccharides applied to the pigs for experimental purpose.

To put it another way, the pathological improvement food of the invention, which chiefly contains indigestible polysaccharides, can take various form such as drink, cream, biscuit, bread, cake, ice cream and provide various textures by diversely combining different kinds of indigestible polysaccharides or determining the amount of each ingredient, or the pathological improvement food of the invention may be made in the form of powder composed of required ingredients. Therefore, desirable diet food as mentioned above may be made in the home according to the patients' predilection and used as recipe ingredients.

The typical of indigestible polysaccharides is pectine. The pectine is contained in plenty in an apple and citrus fruit and remains in existence in high concentration in fruit juice lees. Thus, the pectine can be extracted from the lees and processed into a dry product upon being de-estenfied with acid or alkali and purified. It may also be processed in such a manner of being extracted from the apple, pericarp of citrus fruit or vegetable, de-esterified with acid or alkali, filtrated, concentrated, precipitated with alcohol to be cleansed, dried and broken into powder. The pectine may be taken out by being refined for use in this invention in the manner described above, but apple juice lees or fruit juice lees of citrus fruit (mandarin orange, orange, gapefruit, etc.) may be used as they are for the same purpose as the refined pectine because these juice lees are rich in pectine. That is, the pectine termed in this invention includes the apple juice lees or citrus fruit juice lees. Likewise, alginic acids and fucoidan, which are made from seaweed, and polydextrose made from glucose as a basic ingredient in the presence of citric acid can be used as the indigestible polysaccharide. These indigestible polysaccharides for use in the invention should not necessarily be understood as being limited to the refined products as long as the effect of the invention is not ruined.

The polysaccharide becomes viscous when dissolving in water because it can absorb large volume of water into its polymer structure. Therefore, when the indigestible polysaccharide is used in the preparation of a beverage, the concentration thereof is desirably 0.5% to 30% by weight. Also, it is preferable to determine the concentration of the same to 2% to 30% by weight for creamy food, 2% to 30% by weight for jellylike food, and 5% to 60% by weight for cakes, and 5% to 60% by weight for dried foods such as biscuits.

A patient with kidney failure or hepatic failure often lacks ingestion of trace metals, which are considered to be fundamental to maintenance of physical functioning, such as Mg, Zn, Fe, Cu, Co, Mn, Cr and Se due to unbalanced diet. Likewise, a patient undergoing hemodialysis treatment often lacks ingestion of the trace metals due to exteriorize thereof during hemodialysis, consequently to possibly cause renal anemia.

The pathological improvement food of the invention may contain 0.01% to 5.0% by weight of trace metals in a free state or in the additive form of metal-containing yeast (e.g. "Mineral Yeast" made by Oriental Yeast Co., Ltd.) The pathological improvement food thus produced even containing such trace metals does not have a disagreeable smell endemic to metal nor nasty taste and can be made in safety. The supplementation of trace metal compensates deficiency of the patient and activates the intestinal bacteria. Thus, the pathological improvement food composed chiefly of the indigestible polysaccharides according to the invention can be furher elevated in availability. To be more specific, the ingestion of the indigestible polysaccharide contributes to reduce the level ofblood urea and low-molecular nitrogen compounds such as ammonia, consequently to promote appetite suppression and eliminate a lowering factor for metabolic activity. In addition to the aforesaid prominent effects of the pathological improvement food of the invention, the essential nutrients such as the trace metals noted above are efficiently added thereto, consequently to produce a synergistic effect for improving the physical conditions.

There may be further added lactobacillus spore, e.g. LACRIS (trade name), made by Sankyo Lifetech Co., Ltd., to the aforesaid food in order to stimulate growth of lactic acid bacilli.

The present invention provides effective means capable of decreasing the amount of nitrogen reabsorbed from the intestine into the bloodstream to suppress the rise of concentration of the low-molecular-weight nitrogen-containing compounds in the blood through the exquisite biodynamic mechanism in which the low-molecular-weight nitrogen-containing compounds such as urea discharged from the blood (through the blood vessel) into the intestine are effected for protein synthesis of intestinal bacteria and passed out with feces. To accomplish the intended means, the food prepared with the indigestible polysaccharide, which is not easily exploited by digestive enzyme but is effective for the intestinal bacteria, thereby to make use of its resultant energy for protein synthesis of intestinal bacteria.

Intrinsically, the low-molecular-weight nitrogen-containing compounds in the blood such as urea and ammonia are secreted into the intestine with the bodily fluid and reabsorbed, thus being circulated between the fluid in the intestine and the blood Therefore, the nitrogen-containing low-molecular substances secreted into the intestine can be utilized as a nitrogen source working on the protein synthesis of intestinal bacteria and discharged to the outside of the body as feces upon proliferating the fungus bodies, thereby to break off the circulation of nitrogen-containing compounds between the intestine and the blood, resulting in a lower concentration in the blood. Thus, the excretion of the compounds effects alleviating of a burden on the kidney.

The reduced burden of the kidney causes degeneration of kidney glomerulus to be retarded, so as to have an expectancy to retard a transition to hemodialysis treatment and decrease the number of hemodialysis.

If a kidney failure patient periodically takes the pathological improvement food of the invention after each meal and/or between meals, a part of the low-molecular-weight nitrogen-containing compound in the blood is partially converted into intestinal bacterial protein and then excreted as feces, resulting in lowering of the blood concentration thereof, so as to enable the transition to hemodialysis treatment to be retarded and the number of required hemodialysis to be decreased. As a result, the various problems posed by the hemodialysis treatment can effectively be solved, consequently to heighten the quality oflife of the patient.

Also, when the pathological improvement food having the function of driving the fungal protein synthesis of intestinal bacteria with ammonia secreted into the intestine and excreting the ammonia with feces is given to a patient with hepatic failure after each meal and/or between meals, the problems as above can be solved, so that distress involved in taking high doses of adsorbent agent can be relieved without producing any side effect according to the invention.

The nitrogen-containing compound, specifically ammonia, in the urine, emits urine odor, whereas the urine odor given out due to anesthesia excretion of a bedridden patient or elderly person can be diminished.

The present invention will be described hereinafter in more detail, demonstrating some medicalizing cases using the pathological improvement food of the invention, but the invention does by no means contemplate imposing any limitation to the cases and compositions described below

### (Embodiment 1) Beverage

Table 1 below shows solid contents of the essential constituent parts of the pathological improvement food of the invention per 100 grams of beverage made by adding water to the essential constituents. There was prepared 100 grams of beverage (product) by adding water thereto.

**TABLE 1**

| Basic Ingredients | Solid contents per 100 grams of beverage |
|---|---|
| Polydextrose | ~20g |
| Pectine | ~20g |
| Fructose | ~5g |
| Citric acid - Na | ~400mg |
| Vitamin C | ~500mg |
| Vitamin B₁ | ~20mg |
| Vitamin B₂ | ~20mg |
| Vitamin B₆ | ~2mg |
| Vitamin B₁₂ | ~1mg |
| Nicotinio-acid amide | ~20mg |
| Folic acid | ~5mg |
| DHA | ~0.5g |
| Calcium glycerophosphate | ~300mg |
| MgCl₂ | ~200mg |
| Fe-containing mineral yeast | ~30mg |
| Zn-containing mineral yeast | ~50mg |
| Cu-containing mineral yeast | ~30mg |
| Mn-containing mineral yeast | ~10mg |
| Arum root powder or Agar | q.s. |
| Food flavor (of apple, citrus and so on) | q.s. |
| Fruit juice or fruit essence | q.s. |
| Texture improving agent | q.s. |
| Sweetener | q.s. |

| | |
|---|---|
| Water is added to define the total as 100. | |
| pH: 45 to 5.5 / Viscosity: 50 to 500 mPas | |
| Color: Light yellow -light brown / Taste: Moderately adjusted with sweet and acid | |
| Energy: 30 to 80 kcal/100g | |
| Mineral Yeast: Cr- or Se-containing mineral yeast may be added arbitrarily. | |

Table 1 exemplifies the daily intake of the beverage. The beverage may be taken in daily after each meal during dialysis treatment at one time or in divided doses. Alternatively, it may be drunk with food items described in Embodiment 2 and Embodiment 3 in combination. The daily intake as exemplified herein may of course be increased, but calorie of the food according to the invention may be preferably reduced to not exceeding 300 kcal per day for example. Particularly in a case of a diabetic patient on dialysis, the caloric intake by the nutraceutical food of the invention should be reduced as low as possible to lessen the influence on calorie count of essential staple food

Apple juice lees can be substituted for pectine. The pectine derived from apple juice, strawberry, citrous or other fruits or pectine-containing foodstuff may be used as an alternative thereto. Also, there may be used other ingredients than pectine, such as acacia gum falling into indigestible polysaccharides, testa-derived hemicellulose, seaweed-derived alginic acids, arum root-derived mannan and so forth instead. The pectine may be displaced in part.

Fructose may be replaced with one of sucrose, sugar alcohol and other sweeteners or a mixture of these. As the sweetener, there may also be used natural sweetener such as luohan fruit essence, stevia, licorice extract. An artificial sweetener such as Aspartame may be used

As to vitamins, there are added vitamin C that is easier to lack and vitamin B family that is losable during the hemodialysis treatment In specific, since minerals such as trace minerals (Fe, Zn, Cu, Mn, Cr, Se, etc.) essential for maintenance of body function have not been approved as food additives by Ministry of Health, Labour and Welfare, yeast containing some of these ingredients is added by the quantity required for daily requirement for adult for nutritional considerations.

For improving the texture of the pathological improvement food, 0.1 to 3 grams of agar powder or aliment arum root powder may be added to make the food jelly.

When drinking 200 to 300 grams (equivalent in solid content) ofthe beverage thus prepared a day, 4 to 8 grams of protein taken on the same day are fixed into intestinal bacterial protein, consequently to be excreted with feces. As a result, the blood concentration of low-molecular-weight nitrogen-containing compounds such as urea can be decreased corresponding to the amount of nitrogen content in the fungal protein, to alleviate a burden on the kidney.

The conditions at that time can be formulated by the following formula.

That is, there can be proved experimentally the following relation in which the intestinal bacteria gains energy by exploiting indigestible polysaccharides using, as a nitrogen source, low-molecular-weight nitrogen-containing compounds such as urea secreted in the intestine in fungal protein synthesis.
(a) Assuming that the total energy content of indigestible polysaccharide is generally 3.6 kcal/g and 50% of the energy is consumed by the fungus body, the energy at which the indigestible polysaccharide (Ag) are consumed by the fungus body is Ag×3.6 kcal/g × 0.5 = 1.8 × A(kcal).
(b) Assuming that the energy required for fungal protein synthesis is generally 5.66 kcal/g protein and the energy use efficiency by the intestinal bacteria is 44%, the fungal protein synthesis of Bg necessitates Bg × 5.66 kcal × 0.44 = 12.8 × B(kcal). Accordingly, the approximate amount of bacterial protein (Bg) synthesized by oral administration of the indigestible polysaccharide of Ag is 1.8×A/12.8.
(c) When taking 300 g of beverage of Embodiment 1 noted above a day, it corresponds to the intake (A) of 30 to 60 grams of the indigestible polysaccharides, and the fungal protein content (B) synthesized at that time becomes 4.2 to 8.4 grams.

When a kidney failure patient (50 kg body weight) consumes 30 grams of protein a day (0.6 g/kg body weight), the intake of 300 grams of the beverage in terms of solid content brings about the same effect as intake reduction by 4% to 17% of protein.

### (Embodiment 2) Jellylike food

Table 2 below shows parts ofthe essential constituents of the jellylike food.

**TABLE 2**

| Basic Ingredients | Blending Quantity |
|---|---|
| Apple juice lees | 3 to 20 g |
| Pectine | 1 to 10 g |
| Polydextrose | 10 to 20 g |
| Arum root-derived mannan | 0.1 to 2 g |

| | |
|---|---|
| Water is added to be 60 to 105 grams. | |

Fructose, citric acid, sodium citrate, vitamin C, vitamin B family (B1, B2, B6, nicotinio-acid amide, and B12), trace metals, and mineral yeast all are contained in a similar manner in the beverage (former embodiment) and the jellylike food. Thus, the jellylike food can be produced by adding fructose and the subsequent ingredients shown in Table 1 to three ingredients shown in Table 2. The water content of the food is regulated by adding water to the food so as to make a total of 60 to 105 grams in total.

The jellylike food prepared in the aforementioned manner may be stored in containers of, for example, 20, 25 or 35 grams in a sterile condition. It may of course be contained in a larger container or packager.

The aforesaid jellylike food (20-gram, 25-gram or 35-gram package) may be taken after each meal or during dialysis treatment as needed. When consuming one dose of food of the quantity described above three times a day, the daily intake of indigestible polysaccharides becomes roughly 25 grams (20-gram package) to 40 grams (35-gram package), so that 3.5 grams/day (20-gram package) to 6.3 grams/day (35-gram package) of protein ingested from the food on the day are fixed onto the intestinal bacterial protein and excreted into feces.

Even if the protein intake (or nitrogen intake) fluctuates from day to day, it is equivalent to the total excretion amount of nitrogen. The intake of indigestible polysaccharides enhances the effect of introducing the nitrogen into feces via the principal excreting route rather than feces routed through the kidney or large intestine, thereby to lessen a burden imposed on the kidney and bring about an effect of detoxication and exclusion of nitrogen-containing compounds such as urea secreted into the intestine.

Incidentally, a hepatic failure patient may possibly be relieved from the sufferings from gastrointestinal disorder such as diarrhea due to ammonia secreted into the intestine.

### (Embodiment 3) Confectionery such as biscuits or cookies

Dry or semidry products such as cookies, biscuits bread or rice crackers may be produced by using indigestible polysaccharides as a chief constituent. The composition of the chief constituent of food will be shown in Table 3 below

**TABLE 3**

| Basic Ingredients | % (Dry weight) |
|---|---|
| Polydextrose | 5 to 25% |
| Apple juice lees | 10 to 40% |
| Pectine | 3 to 15% |
| Cellulose fiber | 5 to 10% |
| Oligosaccharide | 15 to 30% |
| Starch (Low-gluten wheat) | 5 to 20% |
| Butter (or Shortening) | 10 to 20% |
| Others (*) | 1 to 10% |

| | |
|---|---|
| (*) 1% to 10% of sweetener, food flavor, vitamins and minerals are added on the basis of Embodiments 1 and 2. | |

As the indigestible polysaccharides, there may also be used not merely apple juice lees, but also acacia gum, psyllium and water-soluble indigestible polysaccharides derived from cereal testa such as of soybeans and corn. Bacillus coagulant (e.g. LACROSSE (Trade name)) may be added

As cellulose fiber, microscopic cellulose powder such as "SolcaFloc 300FCC" (Danisco Japan KK) may be used by way of example.

As the starch, potato starch, corn starch, kudzu starch, and low-gluten wheat, which are is low in protein, may be preferably used. Soft wheat flour may be used within the realm of using the starch. In the case of using the apple juice lees in baking the components shown in Table 3 to made the biscuits or cookies, the pectine-rich apple juice lees become like mud and hard to bake particularly when they are enriched with polydextrose and pectine. Consequently, the components containing a large amount of pectine is not suitable for such baked products, but wheat flour may be added so as to be over 20% to the total amount to improve the physical properties of the components. In making the product in this embodiment, low-gluten wheat is preferably used to restrict the protein ingredient to less than its specific quantity while preventing flour-derived protein gluten from admixing.

As to the ingredients of cookie and biscuit, the biscuit contains butter (or shortening) that is smaller in amount than in the cookie, and the cookie and biscuit both contain baking soda and citric acid or sodium citric acid to prevent hardening and thus to provide favorable texture. Although the cookies and biscuits are generally baked by using a common baking device, baking of the cookies and biscuits by microwave heating brings about an effect of releasing moisture content from the dough ingredients to make numerous pores in the products and provide favorable texture.

The butter and shortening are edible oil and fat and solid at room temperature in a lot of cases. The butter and shortening may contain oleic acid, palmitic acid, linoleic acid, linolenic acid and/or fatty acids having a carbon number of 12 or 14. There may be further added polyunsaturated fatty acid such as DHA in order for protecting the central nervous system susceptible to disorder ofthe kidney or liver.

The cookies and biscuits thus produced can ensure protein content of 0.1 to 4 grams per day. The food products of this kind provide 400 kcal per day available to a patient, so that a large amount of indigestible polysaccharide can be given without affecting the dietary schedule for the patient (1800 to 2000 kcal per day), consequently to decrease the blood level of urea or ammonia. Thus, the food products according to the invention provide synergistically therapeutic benefit to nephropathy and hepatic failure.

When taking six articles of food (e.g. cookies) made from the ingredients described above every day, the intake of indigestible polysaccharides is about 30 to 50 grams. As a result, 4 to 6 grams of dietary protein are bypassed through the fecal route.

The food products such as the beverage, jelly and cookies shown in Embodiments 1, 2 and 3 may be taken singly or in combination so as to excrete substantial amount of dietary-derived nitrogen components with urine and feces. The combination of the pathological improvement food of the invention and regular meals including clinical diet brings about a noticeable synergistic effect with ease.

When taking 100 grams of beverage (solid content equivalent), two packages of jelly (25 grams per package), and six cookies by mixture daily in several divided intakes, fungal protein synthesis runs to 10 to 15 grams, resulting in excretion of the low-molecular-weight nitrogen-containing compounds corresponding to equal parts of the fungal protein synthesis through a defecation route, not through a micturition route.

### (Embodiment 4) Other type of food

A diet food may be made by mixing 5% to 30% by weight of indigestible polysaccharides into noodle while reducing starch as flour as small as equivalent to the indigestible polysaccharides. In this case, the amount of indigestible polysaccharides to be added should be adequately determined based on the protein content in the flour or cereal starch so as to moderate the intake of protein to 0.6 g/kg body weight or less or 12 to 1.5 g/kg body weight per day.

The indigestible polysaccharides may be used as the basic ingredients of frozen desserts and confectionery. There may be added 5% to 20% by weight of polydextrose of the indigestible polysaccharides. The semisweet polydextrose is suitable for confectionery. Addition of 1% to 5% of citrus pectine such as of an apple or strawberry gives the confectionery more sweet taste. The quantities of the principle ingredients of the confectionery such as milk and egg or protein components derived therefrom should be moderated to accomplish the objects of the invention. Such being the case, in place of 20% to 70% by weight of protein in the food, there may be used a substituting ingredient made by adding 5% to 30% by weight of soybean or egg-derived lecithin to emulsified soybean made by condensing supernatant fluid obtained in coagulating soybean curd. The protein components derived from milk or egg may be substituted for an ingredient made by adding soybean or egg-derived lecithin to the emulsified soybean to have high affinity with water. Thus, the present invention can provide the pathological improvement food having a noticeable synergistic effect with indigestible polysaccharides by suppressing the protein intake.

### (Embodiment 5) Test for improvement of clinical conditions (Case 1)

Three packages of jelly-like food (25 grams each) as described in Embodiment 2 and six cookies as described in Embodiment 3 were given to four healthy male volunteer subjects and four healthy female volunteer subjects (eight subjects in total) a day. The total quantity of indigestible polysaccharides was 80 grams per day on average, and the daily protein intake was 0.6 g/kg body weight.

The experimental subjects were divided into two groups by gender and subjected to a cross-over method To be specific, two male and two female subjects in one group started eating control diet food at the outset of the dietary test and took experimental diet food from the 5th day, and then, continued to eat the experimental diet food for 4 days. The other group composed of two male and two female subjects started eating the experimental diet food and took and continued to eat control diet food from the 5th day for 4 days.

An explanation about the objectives of the dietary test, the compositions of the food under test and safety of the food was made to the experimental subjects to gain their consent in writing.

Blood samples of the subjects were taken in the morning fasting to measure BUN, and all urine of the respective subjects was collected to provide measurement for urinary nitrogen every day. The experimental results obtained eventually are shown in Table 4 below

**TABLE 4**

| | BUN | Nitrogen Amount/total urine volume/day |
|---|---|---|
| Control Group | 20.8±5.3mg/dl | 12.3±4.5g/day |
| Testing Group | 14.5±6.5mg/dl | 8.1±2.9g/day |

As shown in Table 4, the excretion amount of nitrogen into the urine was decreased by 34% and BUN is improved by 30%.

### (Embodiment 6) Test for improvement of clinical conditions (Case 2)

An experiment on the influence of the pathological improvement food on nitrogen excreted into the feces, BUN, urinary urea and ammonia was conducted by giving three jelly-like food products (25 grams each) as shown in Embodiment 2 a day and six cookie-like food products in two to three divided doses a day.

From kidney failure patients who need not hemodialysis, but have higher BUN and have habitually kidney-supporting diets, five experimental subjects were selected with the written consent of the patients upon explaining the research purposes, prescribed diet food and schedule of experiment. The experiment was conducted by collecting blood samples from the patients at the same time each day to measure BUN and collecting all urine from the respective patients to measure the amounts of urea and ammonia every day All feces of the respective patients were collected on the 7th, 8th and 9th days during the test to measure the total nitrogen amounts. On 7th, 8th and 9th days, the same meals were prepared, and the total amount of nitrogen of each meal and the intake of nitrogen were calculated. The calculated amounts thereof are shown in Table 5 below.

**TABLE 5 (1) Feces**

| Test period | Control period for non-dose | | | Period for dose | | |
|---|---|---|---|---|---|---|
| Lapsed days | 7 | 8 | 9 | 7 | 8 | 9 |
| Fecal amount | 952±328 | 985±29.1 | 103.8±43.7 | 93.7±283 | 1029±402 | 126.1±532 |
| Total nitrogen amount (g/day) | 1.9±0.45 | 2.0±0.21 | 2.1±0.28 | 2.50±0.50 | 2.62±0.45 | 2.80±0.23 |

**(2) Blood and Urine**

| Test period | | Control period for non-dose | | | | | Period for dose | | | | |
|---|---|---|---|---|---|---|---|---|---|---|---|
| Lapsed days | | 1 | 4 | 7 | 10 | 14 | 2 | 4 | 7 | 10 | 14 |
| Blood | BUN (mg/dl) | 35.3 | 362 | 33.7 | 34.6 | 34.8 | 30.8 | 283 | 26.1 | 25.3 | 24.8 |
| Urine | Volume (L/day) | 1.51 | 1.39 | 1.46 | 1.53 | 1.48 | 1.43 | 1.56 | 138 | 1.52 | 1.48 |
| | Total nitrogen (g/day) | 3.7 | 3.9 | 3.9 | 3.4 | 3.6 | 32 | 2.8 | 2.8 | 2.4 | 2.6 |

As seen from Table 5, increase in amount of the feces and nitrogen excreted into the feces was confirmed on 9th day of a course of the experiment by the possible cause considered to be formula of the sample diet food. It was also confirmed that BUN was decreased by 20% and the excretion amount of nitrogen into the urine was improved by 30% to 60% on a daily basis.

### (Embodiment 7) Test for improvement of clinical conditions (Case 3)

From patients undergoing dialysis three times a week, five experimental subjects were selected with the written consent of the patients upon explaining the research purposes, prescribed diet food and schedule of experiment in the same manner as in Embodiment 6. The experiment was conducted by giving the same diet food of the same amount as supplied in Embodiment 6 to the patients, to measure the total amount of nitrogen excreted into the feces and BUN. The results are shown in Table 6 below.

**TABEL 6**

| Test period | Control period for non-dose | | | Period for dose | | |
|---|---|---|---|---|---|---|
| Lapsed days | 7 | 8 | 9 | 7 | 8 | 9 |
| Fecal amount | 75.8±22.8 | 813±29.1 | 78.8±43.7 | 73.7±22.3 | 82.9±30.2 | 86.1±53.2 |
| Total nitrogen amount (g/day) | 130±12 | 1.43±23 | 135±18 | 1.49±29 | 1.62±26 | 157±35 |
| BUN (mg/dl) | 48.5 | 532 | 46.6 | 34.8 | 373 | 32.4 |

As seen from Table 6, increase in amount of the feces and nitrogen excreted into the feces was confirmed on 9th day of a course of the experiment by the possible cause considered to be formula of the sample diet food. It was also confirmed that BUN was decreased by about 30% on the 9th day and significant improvement occurred.

### INDUSTRIAL APPLICABILITY

The pathological improvement food according to the invention has a function of decreasing reabsorption of low-molecular-weight nitrogen-containing compounds such as urea circulating in the blood and intestine and then excreting the nitrogen-containing compounds in the form of feces through the mechanism of using nitrogen of ingested protein as an agent for fungal protein synthesis of intestinal bacteria. Accordingly, the pathological improvement food of the invention brings about the following effects.

(1) The blood concentration of the nitrogen-containing low-molecular substances such as BUN and ammonia can be lowered (2) It becomes possible to alleviate the burdens exerted on the kidney of a kidney failure patient and retard a transition to hemodialysis treatment only by taking a specified quantity of the food of the invention every day. (3) The number of required hemodialysis required for treating kidney failure can be decreased to lessen the psychological, temporal and financial burdens upon the patient and heighten the quality of life of the patient while saving medical expenses. (4) The blood ammonia level can be decreased so as to keep a patient with acute or chronic hepatic failure from a danger of falling into hepatic coma and improve the medical condition ofthe patient

## Claims

1. Pathological improvement food for lowering blood concentration of low-molecular-weight nitrogen-containing compounds, which contains, as basic ingredients, indigestible polysaccharides and has restricted addition of protein components.

2. Pathological improvement food for lowering blood concentration of low-molecular-weight nitrogen-containing compounds, which contains more than 5% by weight of indigestible polysaccharides in terms of dried foodstuff and protein components restricted to 8% or less by weight.

3. Pathological improvement food for lowering blood concentration of low-molecular-weight nitrogen-containing compounds according to claim 1 or claim 2, wherein said indigestible polysaccharides are one kind selected from pectine, polydextrose, alginic acid, fucoidan, chitin, chitosan, testa-derived hemicellulose, acacia gum, arum root-derived mannan, agar, and sugar alcohol and polymers of sugar alcohol.

4. Pathological improvement food for lowering blood concentration of low-molecular-weight nitrogen-containing compounds according to claim 2, wherein said indigestible polysaccharides contains at least polydextrose and pectine with a ratio of 0.05 to 100 parts by weight of pectine to 100 parts of polydextrose.

5. Pathological improvement food for lowering blood concentration of low-molecular-weight nitrogen-containing compounds according to any of claims 1, 2 and 4, further containing at least one of trace metal, vitamin and fat

6. Pathological improvement food for lowering blood concentration of low-molecular-weight nitrogen-containing compounds according to claim 3, further containing at least one of trace metal, vitamin and fat.

7. Pathological improvement food for lowering blood concentration of low-molecular-weight nitrogen-containing compounds according to any of claims 1, 2 and 4, which food is formed in beverage, biscuit, cookie, cake, ice cream, sherbet, bread, noodle or jelly:

8. Pathological improvement food for lowering blood concentration of low-molecular-weight nitrogen-containing compounds according to claim 3, which food is formed in beverage, biscuit, cookie, cake, ice cream, sherbet, bread, noodle or jelly

9. Pathological improvement food for lowering blood concentration of low-molecular-weight nitrogen-containing compounds according to claim 5, which food is formed in beverage, biscuit, cookie, cake, ice cream, sherbet, bread, noodle or jelly.

10. Pathological improvement food for lowering blood concentration of low-molecular-weight nitrogen-containing compounds according to claim 6, which food is formed in beverage, biscuit, cookie, cake, ice cream, sherbet, bread, noodle or jelly.
